(19) Europäisches Patentamt
European Patent Office
Office européen des brevets

(11) EP 1 991 554 B1

(12) EUROPEAN PATENT SPECIFICATION

(45) Date of publication and mention of the grant of the patent:
**25.12.2013 Bulletin 2013/52**

(21) Application number: **07712384.2**

(22) Date of filing: **28.02.2007**

(51) Int Cl.:
***C07F 9/165*** *(2006.01)* ***A61K 31/6615*** *(2006.01)*
***A61P 35/00*** *(2006.01)* ***A61P 29/00*** *(2006.01)*
***A61P 37/00*** *(2006.01)*

(86) International application number:
**PCT/EP2007/051896**

(87) International publication number:
**WO 2007/099117 (07.09.2007 Gazette 2007/36)**

# (54) THIOPYROPHOSPHATE ORGANIC COMPOUNDS, METHOD FOR PREPARING THEREOF AND COMPOSITIONS CONTAINING THEM

ORGANISCHE THIOPYROPHOSPHATVERBINDUNGEN, VERFAHREN ZU IHRER HERSTELLUNG UND ZUSAMMENSETZUNGEN, DIE SIE ENTHALTEN

COMPOSÉS ORGANIQUES DE TYPE THIOPYROPHOSPHATE, MÉTHODE DE SYNTHÈSE DESDITS COMPOSÉS ET COMPOSITIONS LES INCLUANT

(84) Designated Contracting States:
**AT BE BG CH CY CZ DE DK EE ES FI FR GB GR HU IE IS IT LI LT LU LV MC NL PL PT RO SE SI SK TR**

(30) Priority: **01.03.2006 IT MI20060366**

(43) Date of publication of application:
**19.11.2008 Bulletin 2008/47**

(73) Proprietor: **C4T S.C. a.r.l.**
**00133 Roma (IT)**

(72) Inventors:
- **BRECCIA, Perla**
  **00133 Roma (IT)**
- **ANGELI, Francesca**
  **00133 Roma (IT)**
- **COLIZZI, Vittorio**
  **00133 Roma (IT)**
- **PINZA, Mario**
  **00133 Roma (IT)**
- **POCCIA, Fabrizio**
  **00133 Roma (IT)**
- **TOPAI, Alessandra**
  **00133 Roma (IT)**

(74) Representative: **Spadaro, Marco et al**
**Cantaluppi & Partners**
**Viale della Tecnica, 205**
**00144 Roma (IT)**

(56) References cited:
**EP-B1- 1 987 063** **WO-A-03/070921**
**WO-A-2005/102385**

- **PHAN, RICHARD M. ET AL: "Synthesis of (S)-Isoprenoid Thiodiphosphates as Substrates and Inhibitors" JOURNAL OF ORGANIC CHEMISTRY , 66(20), 6705-6710 CODEN: JOCEAH; ISSN: 0022-3263, 2001, XP002432931 cited in the application**
- **ESPINOSA ET AL: "Y2K+1 state-of-the-art on non-peptide phosphoantigens, a novel category of immunostimulatory molecules" MICROBES AND INFECTION, vol. 3, 2001, pages 645-654, XP002432932 cited in the application**
- **MIYAKAWA ET AL: BRAIN RES. BULL., vol. 56, no. 6, 2001, pages 517-519,**
- **'acceptable', [Online] Retrieved from the Internet: <URL:http://www.thefreedictionary.co/acceptable>**
- **'physiological', [Online] Retrieved from the Internet: <URL:http://www.thefreedictionary.co/physio logical>**
- **DATABASE SCIENCELAB.COM MATERIAL SAFETY DATA SHEET: 'Tetrabutylammonium hydroxide'**
- **DATABASE SCIENCELAB.COM MATERIAL SAFETY DATA SHEET: 'Tetrabutylammonium bromide'**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 991 554 B1

## Description

Technical Field

This invention relates to a thiopyrophosphate organic compound, a method for preparing thereof and a composition containing it.

More particularly the thiopyrophosphate organic compound according to this invention has activating activity on γδ T lymphocytes.

Background to the Invention

It is known that some organic compounds of pyrophosphoric acid, both natural and synthetic, have activating activity on γ9δ2 T lymphocytes (Fournié et al., Microbes and Infection 2001, 3, 645-654). Owing to this activity they are useful in the treatment of immunological and allergic disorders (asthma, Chron's disease, multiple sclerosis) (Bendelac et al., Nat. Rev. Immunol. 2001, 1, 177-185) and infectious diseases (Wang et al., J. Clin. Invest. 2001, 108, 1349-1357) and tumours (Girardi et al., Science 2001, 294, 605-609; Sicard et al., Mol. Med. 2001, 7, 711-722).

Moreover U.S. 5,639, 653 describes the use of compounds of formula

$$ROPO(OH)OP(OH)_2$$

where

R is a straight or branched alkyl radical having from 1 to 4 carbon atoms, or a straight or branched alkenyl radical having from 2 to 20 carbon atoms,

to stimulate the proliferation of Vγ2Vδ2 T cells in mammals and in particular for the treatment of lymphomas, leukaemia, leprosy, malaria, AIDS, rheumatoid arthritis, ulcerous colitis and anaemia

It is also known that other organic compounds of pyrophosphoric acid, both natural and synthetic, behave as activators of γδ T lymphocytes. One example of these natural compounds is isopentenyl pyrophosphate (IPP) ("Isopentenyl Pyrophosphate, a Mycoobacterial Non-peptidic Antigen, Triggers Delayed and Highly Sustained Signaling in Human γδ T Lymphocytes without Inducing Down-modulation of T Cell Antigen Receptor" Lafont, V. et al., J. Biol. Chem. 2001, 276, 15961-15967), while of the synthetic compounds known so far the most active appear to be the phosphohalodrins disclosed in U.S. 6,660,723 B1 and Espinosa, E. et al., (J. Biol. Chem. 2001, 276, 18337-18344)

The formulae of IPP and the above mentioned phosphohalodrins are as follows:

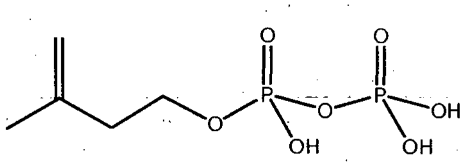

IPP

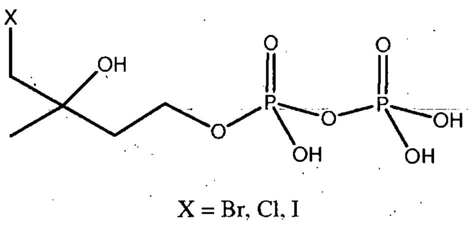

X = Br, Cl, I

Among the natural phosphoantigens initially identified in extracts from *Mycobacterium tuberculosis,* the compound (E)-4-hydroxy-3-methyl-2-butenyl pyrophosphate (an intermediate in the alternative Rohmer metabolic pathway to mevalonate) extracted from *E.coli* is today the most active stimulant of γ9δ2 cells (Hintz, M. et al., Febs. Lett. 2001, 509, 317-322).

As a result of investigations of the relationship between structure and activity in a class of products having γδ T lymphocyte activating activity, it has been reported that two molecular components have to be present: an alkenyl chain and a pyrophosphonic group.

The alkenyl chain associated with the pyrophosphate group showing activating properties on γδ T lymphocytes may have of from 3 to 5 carbon atoms and a double bond in 2 or 3 position (Morita, C. et al., J. Immunol. 2001, 167, 36-41).

In other investigations an increase in activity due to addition of a halohydrin in C3 position or substitution of the double bond between C3 and C4 with a carbonyl group in the C4 position (Belmant, C. et al., FASEB J., 2000, 14, 1669-1670) has been observed. In these investigations it has been shown that it is also necessary for a hydrolysable phosphodiester bond to be present (Belmant, C. et al., FASEB J., 2000, 14, 1669-1670). ,

After activating compounds have been incubated with γδ T cells it has been found that a molecule of free phosphate is produced.

Furthermore, in the literature there have been reported inconsistent data concerning the activity of bisphosphonate compounds in which the P-$CH_2$-P sequence of bonds is present instead of the P-O-P sequence. In fact according to some authors substitution of the P-O-P sequence with the P-$CH_2$-P sequence gives rise to a drastic reduction in the ability to activate γgδ2 T lymphocytes (Bioorg. & Med. Chem. Lett. 13, (2003) 1257-1260). Conversely, according to other authors compounds having the sequence P-$CH_2$-P behave as γgδ2 T lymphocyte inhibitors (Belmant et al., U.S. 6,624,151 B1).

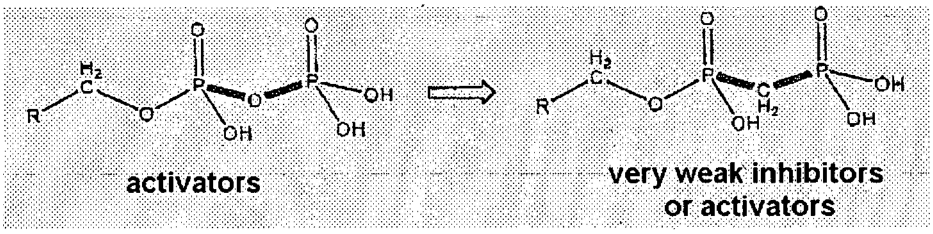


The presence of the sequence C-O-P has also been investigated on account of its limited metabolic stability, which is crucial for possible clinical applications in immunotherapy; structural modifications in which the sequence C-C-P is present instead of the sequence C-O-P have recently been investigated (Zgani, I. et al., J. Med. Chem. 2004, 47, 4600-4612) obtaining compounds in which the mean lifetime increases and preserves the activity of the corresponding metabolite.

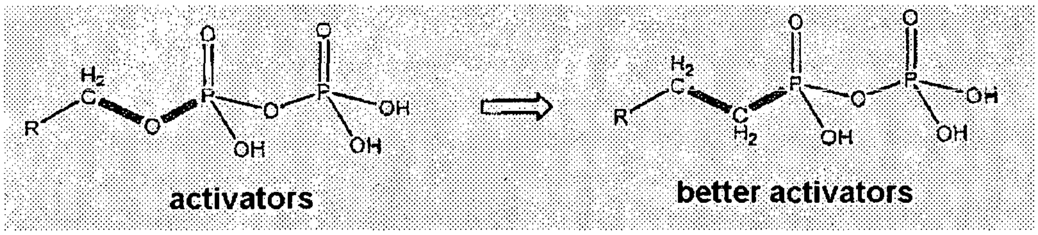


Finally, some compounds of the thiopyrophosphate type which have the sequence C-S-P instead of the sequence C-O-P, analogues of isopentenyl pyrophosphate and derivatives (geranyl, farnesyl pyrophosphates) are also known in the literature, but of these only their activity as inhibitors/substrates for the enzyme farnesyl/geranyl/ undecaprenyl pyrophosphate synthetase have been evaluated (Phan, R.M.; Poulter, D., J. Org. Chem. 2001, 66, 6705-6710). In these experiments the presence of a thiopyrophosphate group instead of a pyrophosphate group converts the substrates (FPP into FsPP) into inhibitors (Chen et al., J. Biol. Chem. 2002, 277, 7369-7376).

It has also been reported in the literature that the conversion of germanyl pyrophosphate into the corresponding thiopyrophosphate confers inhibiting activity on farnesyl pyrophosphate synthetase (FFPase) of which it is a natural substrate (Dale Poulter et al., J. Am. Chem. Soc. 1991, 113, 4895; Organic Letters 2000, 15, 2287).

Antigen activity on γδ T lymphocytes has never been evaluated in these thioderivatives.

Thus as the basis of the molecular mechanism of the γδ TCR-phosphoantigens recognition is still unknown and it has not yet been proven that this direct recognition is really responsible for activation, those skilled in the art do not yet have any means of foreseeing what effect changes in the structure of natural phosphoantigens might have on the function of γδ T lymphocytes.

Now, surprisingly, a class of thiopyrophosphates which are chemically more stable than the corresponding pyrophosphates and which have activating activity on γδ T lymphocytes has been found.

Description of the Invention

In a fist aspect this invention relates to a compound of general formula (I):

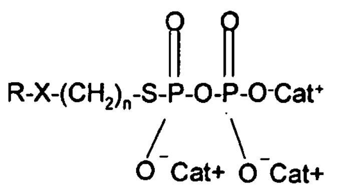
(I)

in which

X is an oxygen atom, a sulphur atom or a covalent bond,
n is zero when X is a covalent bond and is 1, 2 or 3 when X is O or S,
R is

(i) a straight or branched saturated or unsaturated aliphatic group having 1 to 9 carbon atoms, in which one hydrogen atom may be replaced by a halogen atom, a hydroxy group, a nitrile group, a cycloalkyl group having 3 to 6 carbon atoms, an aryl group or a heterocyclic group, or
(ii) a heterocyclic group, and

$Cat^+$ is a physiologically acceptable organic cation selected from lysine, arginina, tromethamine, hydroxyethylpyrrolidine, triethanolamine and N-methylglucamine or a physiologically acceptable inorganic cation selected from alkali metals and alkaline earth metals.

Preferably, one hydrogen atom of said straight or branched saturated or unsaturated aliphatic group having 1 to 9 carbon atoms is replaced by a halogen atom, a hydroxy group, a nitrile group, a cycloalkyl group having 3 to 6 carbon atoms, an aryl group or a heterocyclic group.
Preferred meanings of halogen are chlorine and fluorine.
Preferred meanings of aryl are benzene and pyridine.
Preferred meanings of heterocyclics are oxetane, dioxolane and tetrahydropyran.
Typical examples of inorganic cations are ammonium, alkali metals and alkaline earth metals. Preferred meanings of inorganic cations are $NH_4^+$, $Na^+$ and $K^+$.
Typical examples of organic cations are lysine, arginine, tromethamine, hydroxypyrrolidine, triethanolamine and N-methylglucamine.
The compounds of formula (I) according to the invention activate $\gamma\delta$ T lymphocytes with a potency which is at least similar to that of natural phosphoantigens. They also have the advantage that they are appreciably more stable to enzyme hydrolysys than natural phosphoantigens.
This stability has been proved in an experiment in which the concentration of a IPP compound (isopentenyl pyrophosphate) and Compound 8 of this invention (isopentenyl thiopyrophosphate) in buffered solutions in the presence of alkaline phosphatase (Example 17) was monitored over time.
This makes the compounds of formula I according to this invention metabolically more stable and confers major therapeutic advantages upon them.
In a second aspect, this invention relates to a method for preparing a compound of formula (I) in which n, X, R and $Cat^+$ have the above mentioned meanings, characterised in that an alcohol of formula (II)

$$R\text{-}X\text{-}(CH_2)_n\text{-}OH \qquad (II)$$

in which n, X and R have the above mentioned meanings, is reacted in solid phase with a thiopyrophosphate of formula (III)

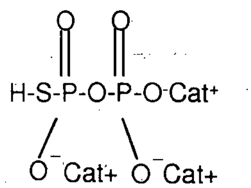

(III)

in which $Cat^+$ has the above mentioned meanings.
Preferably $Cat^+$ is a cation which is capable of solubilizing the compound (III) in organic solvents.
Typically $Cat^+$ is a tetraalkyl ammonium group of formula $(R'')_4N^+$ where R" is an alkyl having 1 to 5 carbon atoms.
A preferred meaning of R" is butyl.
The compound of formula (III) can be prepared according to known methods, such as, for example, the procedure described by Poulter, D.C. and Phan, R.M. (Organic Letters 2000, 2, 2287-2289) which result in the desired compound in 4 stages as shown in Scheme 1 below:

Scheme 1

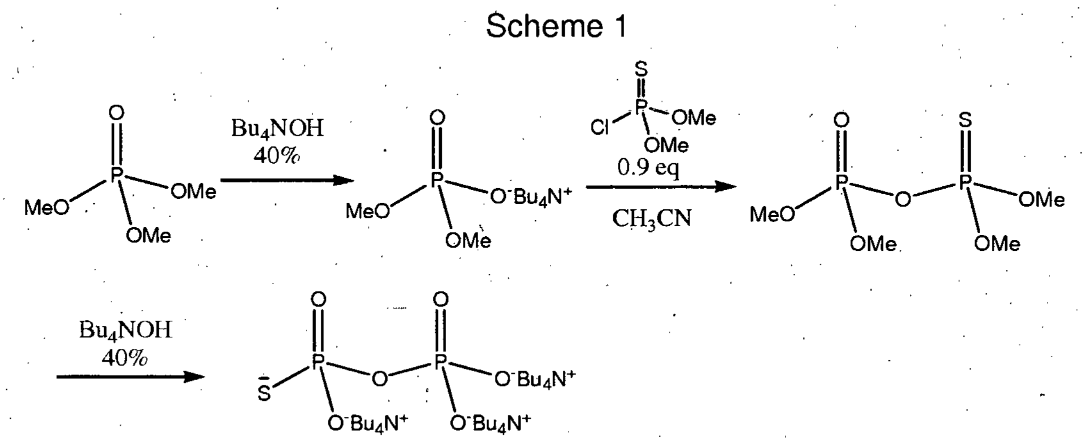


Preferably the solid support used to carry out the reaction in solid pase according to this invention is polystyrene benzene sulphonyl chloride (PS-TsCl) having a low loading capacity-(1.3 mmol/g).

Preferably the equivalents of the alcohol of formula (II) which are bound to the solid support according to the method of this invention range from 1 to 5 depending upon the nature of the R group.

In a preferred embodiment the alcohol of formula (II) is loaded onto the solid support in dichloromethane in the presence of pyridine or dimethylaminopyridine.

**[0037]** - Advantageously the reaction time for the alcohol of formula (II) with the solid support ranges from 24 to 48 hours. Typically the progress of this reaction is monitored qualitatively by the bromophenol blue test, in which a small amount of resin is taken and 1,2-diaminoethane (5% solution in dimethyl formamide) is added thereto and binds to the sites on the resin which have not yet reacted. The resin is then washed and reacted with a solution of bromophenol blue which reacts with the amine groups. The blue colour of the resin pearls therefore shows that loading with the alcohol of formula (II) is not complete and that further alcohol of formula (II) must be loaded.

When the reaction is complete the excess of reactants is removed by filtration and subsequent washings. Advantageously these washings are carried out with dichloromethane, dimethyl formamide, 3:1 tetrahydrofuran/water and finally tetrahydrofuran in succession.

A solution of thiopyrophosphate of formula (III) is then added to the solid support to which the alcohol of formula (II) had been bound.

In a preferred embodiment the solvent is acetonitrile.

Advantageously the amount of thiopyrophosphate of formula (III) added to the solid support having the alcohol of formula (II) bound thereto ranges from 0.8 to 1 equivalent.

Typically this stage is performed under stirring, for a time of approximately 24 hours at ambient temperature.

When the reaction is complete the filtrate is collected and the thus obtained product is converted into the ammonium or sodium salt thereof with an ion exchange resin (DOWEX® 50-WX8-200) in a suitable form ($NH_4^+$ or $Na^+$ form).

The thus obtained products are analysed by LC-MS (negative or positive ESI) and are purified if necessary by precipitation and/or chromatography on a cellulose column.

As an alternative to the solid phase synthesis, the compounds of formula (I) of this invention may also be prepared in solution (see Examples 7, 8 and 10-16) in a manner similar to that described by Poulter (J. Org. Chem. 2001, 66, 6705-6710).

The products obtained in this way are however less pure than those obtained in solid phase and require further purifications.

In a third aspect this invention relates to a pharmaceutical composition comprising a compound of formula (I) of this invention and at least a physiologically acceptable carrier.

In literature, it has been reported that γδ T lymphocytes show cytotoxic activity in response to cells infected with HIV virus (Poccia, F. et al., J. Immunol. 159, 6009-6017; Wallace, M. et al., Clin. Exp. Immunol., 103, 177-184; Poccia, F. et al., J. Infect. Dis. 180, 858-861; Agerberth, B. et al., Blood 96, 3086-3093) and show killer activity towards *Mycobacterium tuberculosis* (Dieli, F. et al., J. Infect. Dis. 184, 1082-1085; Dieli F. et al., Eur. J. Immunol. 30, 1512-1519) and to a number of myelomas and lymphomas (Wihelmm, M. et al., Blood 102, 200-206; Kunzmann, V. et al., Blood 96, 384-392; Fisch, P. et al., Eur. J. Immunol. 27, 3368-3379; Sicard, H.T. et al., Mol. Med. 7, 711-722).

Typical examples of pathological conditions which may derive benefit from treatment with a pharmaceutical composition according to this invention are therefore infections with HIV virus, infections with *Mycobacterium tuberculosis*, myelomas, lymphomas and some kinds of tumour such as kidney carcinoma (Viey, E. et al., J. Immunol. 174 (2005)

1338-1347).

Preferably the pharmaceutical compositions according to this invention are prepared in the form of suitable dosage forms comprising an effective dose of at least one compound of formula I and at least one physiologically acceptable inert ingredient suitable for oral, rectal, topical, intravenous, subcutaneous, intramuscular or intraperitoneal administration.

Examples of suitable dosage forms are tablets, capsules, coated tablets, granules, solutions and syrups for oral administration, medicated creams, ointments and plasters for topical administration, suppositories for rectal administration and sterile solutions for administration by injectable, aerosol or ophthalmic route.

The dosage forms may also contain other conventional ingredients such as: stabilising preservatives, surfactants, buffers, salts to regulate osmotic pressure, emulsifiers, sweeteners, colouring agents, flavourings and the like.

If required by particular treatments the pharmaceutical composition of this invention may contain other pharmacologically active ingredients which it is useful to administer at the same time.

The amount of compound of formula I in the pharmaceutical composition of this invention may vary over a wide range depending upon known factors such as, for example, the nature of the disease to be treated, the severity of the disease, the patient's body weight, the dosage form, the selected administration route, the number of administrations per day and the efficacy of the selected compound of formula I. However, the optimum amount may be determined easily and routinely by those skilled in the art.

Typically the amount of compound of formula I in the pharmaceutical composition of this invention is such as to ensure a level of administration of from 1 to 500 mg/kg/day.

The dosage forms of the pharmaceutical composition of this invention may be prepared according to techniques which are well known to pharmaceutical chemists, which include mixing, granulation, compression, dissolution, sterilisation and the like.

In a fourth aspect this invention relates to a method for activating γδ T lymphocytes in mammals comprising contacting said lymphocytes with an effective amount of a compound of formula I.

Typically the said method is used for the treatment of human patients affected by HIV virus infections, *Mycobacterium tuberculosis* infections, myelomas, lymphomas and some forms of tumours such as kidney carcinoma.

Advantageously the said method comprises sampling a suitable amount of peripheral blood from a patient, contacting the said blood with a compound of formula I according to this invention for a sufficient time to stimulate the *ex vivo* proliferation of γδ T lymphocytes; collecting the thus treated γδ T cells and intravenously administering the same to the patient.

Experimental Part

The examples which follow describe the preparation of some compounds of formula I according to this invention (Examples 1-16), the results of comparative tests (i) of stability to enzyme hydrolysis (Example 17) and (ii) the biological properties of compounds of formula I of this invention in comparison with the most representative natural γδ T lymphocyte activator (IPP) (Example 18).

The analytical characterisations of the compounds have been carried out by means of a HPLC-MS Nebula system (Gilson-Thermofinnegan), a Synergy Polar RP column (150 x 4.6; Phenomenex) or ODS3 column (150 x 4.6; GL Science) and nuclear magnetic resonance spectroscopy ($^1$H-NMR).

$^1$H-NMR spectra were recorded by means of a Bruker Avance 300 MHz instrument.

Example 1

S-(4-chlorobutyl) ester of the trisodium salt of thiodiphosphoric acid

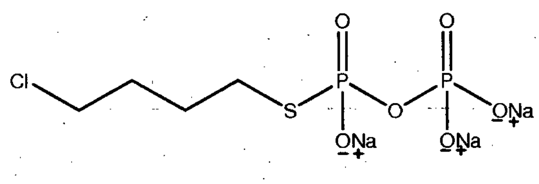

Compound 1

27 mg of 4-chlorobutanol (0.248 mmol) in 0.6 ml of dichloromethanepyridine (1:1) were added to 114 mg (0.165 mmol) of PS-TsCl resin (1.47 mmol/g) swelled in THF.

The reaction mixture was kept under stirring for 24 hours, the excess of reactant was filtered off and the resin was washed with dichloromethane (3 x 1 ml), dimethyl formamide (3 x 1 ml), 3:1 tetrahydrofuran/water (3 x 1 ml), and tetrahydrofuran (3 x 1 ml) in succession. This resin was monitored with the bromophenol blue test until the blue colour of the resin disappeared.

Afterwards the resin was re-swelled in THF and tetrabutylammonium thiopyrophosphate (152 mg, 0.165 mmol) dissolved in acetonitrile (0.6 ml) was added. The reaction mixture was allowed to stand under stirring for 24 hours. The reaction mixture was then filtered and the filtrate containing the product was passed over a DOWEX® ($NH_4^+$) resin to convert the tetrabutylammonium salt into ammonium salt by eluting with a mixture of 2% of 25 mmol $NH_4HCO_3$ in isopropanol (iPrOH).

The product obtained by lyophilisation of the eluate was purified of possible inorganic monothiophosphates or thiopyrophosphates by precipitation of these impurities with an iPrOH:$CH_3CN$:$NH_4HCO_3$ (0.1 M) mixture (4.5:2.5:3). After centrifuging the supernatant was recovered and again lyophilised.

To complete the purification, the product obtained from lyophilisation was further purified by chromatography on cellulose using an iPrOH:$CH_3CN$:$NH_4HCO_3$ (0.1 M) mixture (4.5:2.5:3.) as an isocratic eluent. After lyophilisation the product (32 mg, yield: 61 %) was characterised by LC-MS and $^1H$ -NMR analysis.
Analysis ($ESI^-$) = 283.2 (M-1).
$^1H$-NMR (300 MHz, $D_2O$): $\delta$ (ppm) 3.70 (2H, m), 2.92-2.85 (2H, m), 2.01-1.80 (4H, m).

Example 2

S-[2-(2-chloroethoxy)ethyl] ester of the trisodium salt of thiodiphosphoric acid

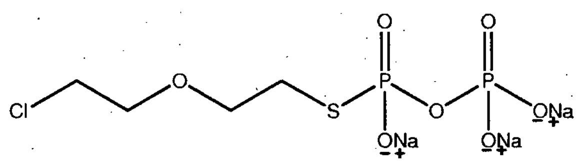

Compound 2

Working in a way similar to that described in Example 1, 90 $\mu$l of 2-(2-chloroethoxy)ethanol (0.850 mmol) in 0.6 ml of dichloromethane (DCM)-pyridine (1:1) were added to 116 mg (0.170 mmol) of PS-TsCl resin (1.45 mmol/g) in tetrahydrofuran (THF).

The reaction mixture was allowed to stand under stirring for 24 hours, the excess reactant had been filtered off and the resin was washed with DCM (3 x 1 ml), dimethyl formamide (DMF) (3 x 1 ml), 3:1 THF/water (3 x 1 ml) and THF (3 x 1 ml), in succession.

When the bromophenyl blue test was positive, this resin was re-swelled in THF and then tetrabutyl ammonium thiopyrophosphate (155 mg, 0.17 mmol) dissolved in acetonitrile (0.6 ml) was added.

After 24 hours the reaction mixture was filtered and the filtrate was passed over DOWEX® resin (NH4$^+$) for conversion into ammonium salt (eluent 25 mM of 2% $NH_4HCO_3$ in iPrOH). After lyophilisation the thus obtained product was purified from possible inorganic monothiophosphates and thiopyrophosphates by precipitating them out with an i-PrOH:$CH_3CN$:$NH_4HCO_3$ (0.1 M) mixture (4.5:2.5:3).
Analysis ($ESI^-$) = 299.1 (M-1).
$^1H$-NMR (300 MHz, $D_2O$): $\delta$ (ppm) 4.05-3.95 (4H, m), 3.92-3.84 (2H, m), 3.24-3.12 (2H, m).

Example 3

S-(2-cyclopropylethyl)ester of the trisodium salt of thiodiphosphoric acid

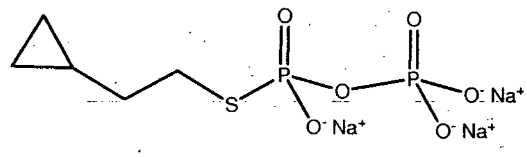

Compound 3

Working in a similar way to that described in Example 1, 71 mg of 2-cyclopropylethanol (0.825 mmol) in 0.6 ml of DCM-pyridine (1:1) were added to 114 mg (0.165 mmol) of PS-TsCl resin (1.450 mmol/g) in THF.
Then 151.3 mg (0.165 mmol) of tetraburyl ammonium thiopyrophosphate (155 mg, 0.17 mmol) dissolved in acetonitrile (0.6 ml) were added. Purification, carried out by precipitation and chromatography on a cellulose column, yielded 40 mg of Compound 3 (yield: 77%).
Analysis (ESI-) = 261.2 (M-1).

Example 4

S-[2-(allyloxy)ethyl]ester of the trisodium salt of thiodiphosphoric acid

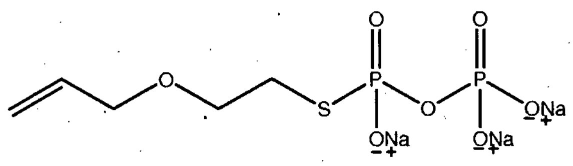

Compound 4

Working in a manner similar to that described in Example 1, 160 μl of 2-(allyloxy)ethanol (1.5 mmol) in 0.8 ml of DCM-pyridine (1:1) were added to 206 mg (0.300 mmol) of PS-TsCl resin (1.45 mmol/g) in THF. 275.4 mg (0.3 mmol) of tetrabutylammonium thiopyrophosphate dissolved in acetonitrile (0.8 ml) were then added. The filtrate was passed over an ion exchange column (DOWEX®-$NH_4^+$ resin). Purification was carried out by chromatography on a cellulose column with $NH_4HCO_3$ (25 mMol):iPrOH:$CH_3CN$ eluent (1:2:1).
After lyophilisation 26.7 mg of Compound 4 were obtained (yield: 30%).
Analysis (ESI$^-$) = 277.2 (M-1)
$^1$H-NMR (300 MHz, $D_2O$): δ (ppm) 6.18-6.04 (1 H, m), 5.48 (1 H, d), 5.38 (1 H, d), 4.22 (2H, d), 3.92 (2H, t), 3.62 (1H, d), 3.21-3.10 (2H, m).

Example 5

S-[(3-methyloxyethane-3-yl)methyl]ester of the trisodium salt of thiophosphoric acid

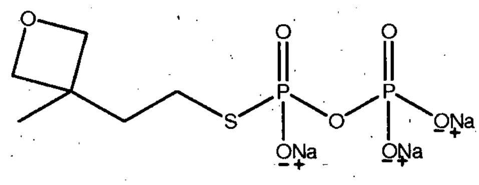

Compound 5

Working In a manner similar to that described in Example 1, 144.5 μl of (3-methyloxyethane-3-yl)-methanol (1.5 mmol) in 0.8 ml of DCM-pyridine (1:1) were added to 200 mg (0.29 mmol) of PS-TsCl resin (1.45 mmol/g) in THF.
293 mg (0.32 mmol) of tetrabutylammonium thiopyrophosphate dissolved in acetonitrile (0.8 ml) were then added. The filtrate was passed over an ion exchange column (DOWEX® $NH_4^+$ resin). Purification was carried out by

chromatography on a cellulose column with $NH_4HCO_3$ (25 mMol):iPrOH:$CH_3CN$ eluent (1:2:1).

88.2 mg of Compound 5 were thus obtained (yield: 92%).

Analysis (ESI$^-$) = 291.2 (M-1)

$^1$H-NMR (300 MHz, $D_2O$): δ (ppm) 4.80 (2H, d), 4.55 (2H, d), 3.27 (2H, d), 1.51 (3H, s).

Example 6

S-(3-hydroxybutyl)ester of the trisodium salt of biodiphosphoric acid

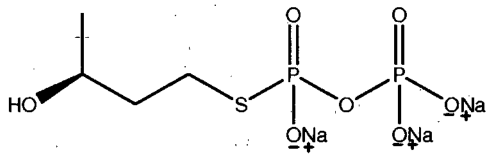

Compound 6

Working in a manner similar to that described in Example 1, 52 μl of S-(3-hyroxy)butanol (0.6 mmol) in 0.8 ml of a solution of DCM and dimethylaminopyridine (DMAP) (35.4 mg, 0.29 mmol) were added to 200 mg (0.29 mmol) of PS-TsCl resin (1.45 mmol/g) in THF.

After 24 hours 293 mg (0.32 mmol) of tetrabutylammonium thiopyrophosphate dissolved in acetonitrile (0.8 ml) were added. The filtrate was passed over an ion exchange column (DOWEX® $NH_4^+$ resin). Purification was carried out by chromatography on a cellulose column with $NH_4HCO_3$ (25 mMol):iPrOH:$CH_3CN$ eluent (1:2:1).

79 mg of Compound 6 were thus obtained (yield: 86%).

Analysis (ESI$^-$) = 279.0 (M-1)

$^1$H-NMR (300 MHz, $D_2O$): δ (ppm) 4.12-4.07 (1 H, m), 3.07-2.97 (1 H, m), 2.90-2.84 (1 H, m), 1.98-1.85 (2H, m), 1.30 (3H, d).

Example 7

S-(benzyl)ester of the trisodium salt of thiophosphoric acid

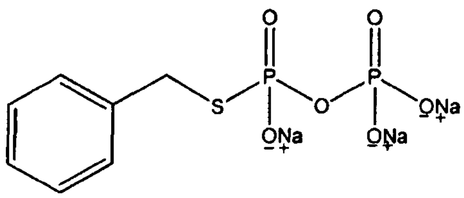

Compound 7

Tetrabutylammonium thiopyrophosphate (63 mg, 0.067 mmol) dissolved in 0.5 ml of acetonitrile was added at 0°C to a 3.56 μl (0.03 mmol) solution of benzyl bromide in acetonitrile (0.5 ml). The reaction mixture was kept under stirring at room temperature for 3 hours.

After removal of the solvent by evaporation at reduced pressure the crude material was purified as described in Example 1 (DOWEX® $NH_4^+$ ion exchange column, precipitation and chromatography on a cellulose column) to yield 8.2 mg of a pure product (yield: 81%).

Analysis (ESI$^-$) = 283.2 (M-1)

$^1$H-NMR (300 MHz, $D_2O$): δ (ppm) 7.50 (2H, d), 7.42 (2H, t), 7.37-7.33 (1 H, m), 4.13 (2H, d).

Example 8

S-(3-methyl-3-butenyl)ester of the triammonium salt of thiodiphosphoric acid

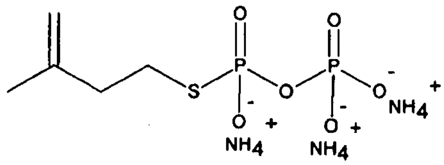

Compound 8

Working in a manner similar to that described by Poulter in J. Org. Chem. 2001, 66, 6705-6710, 11.1 mg of isopentenyl tosylate (0.046 mmol) and 100 mg of tetrabutylammonium thiopyrophosphate (0.106 mmol) were used. After purification (NH4+ ion exchange column, precipitation and chromatography on a cellulose column) 7.1 mg of Compound 8 were thus obtained (yield: 49%).
Analysis ($ESI^-$) = 261.0 (M-1)
$^1$H-NMR (300 MHz, $D_2O$): $\delta$ (ppm) 4.85 (2H, d), 3.04-2.97 (2H, m), 2.44 (2H, t), 1.77 (3H, s).

Example 9

S-[(*E*)-4-hydroxy-3-methyl-2-butenyl]ester of the trisodium salt of thiodiphosphoric acid

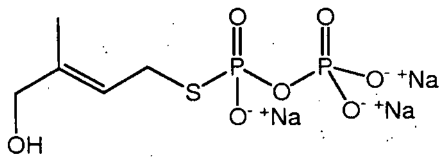

Compound 9

Compound 9 was obtained by working in a way similar to that described by Amslinger et al. (J. Org. Chem. 2002, 67, 4590) except that tetrabutylammonium thiopyrophosphate was used instead of tetrabutylammonium pyrophosphate and the deprotection of the alcohol group was carried out in a different way (see Scheme 2):

Scheme 2

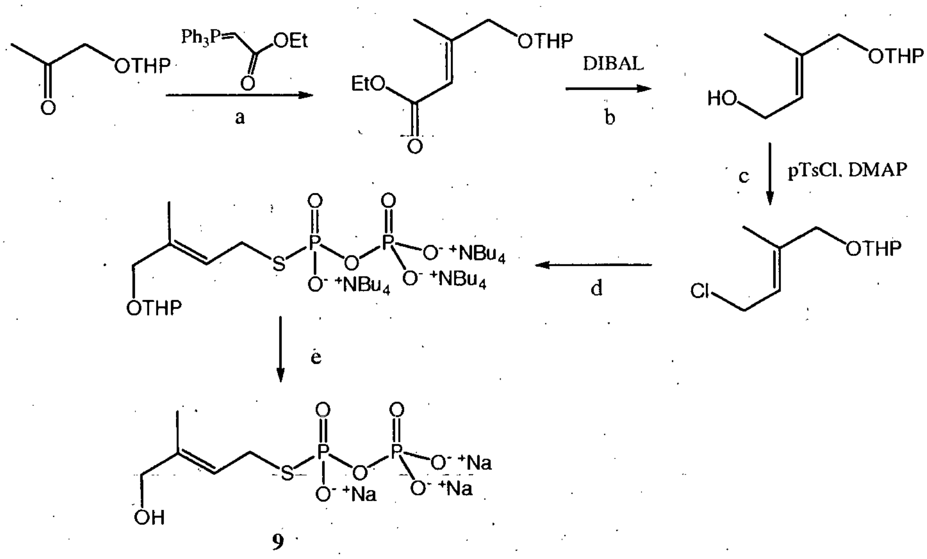


9

(a) toluene, reflux (23 hours),

(b) (1) DIBALH, $CH_2Cl_2$, -78°C (2.5 hours),

(b) (2) 1 M NaOH/$H_2O$,

(c) p-TsCl, DMAP, $CH_2Cl_2$, 25°C (3 hours),

(d) tris(tetra n-butylammonium) hydrogen diphosphate, $CH_3CN$, 25°C (3 hours),

(e) $NaCNBH_3$, $BF_3OEt_2$, 25°C (20 min).

After purification on an ion exchange column (eluent: 2% 25 mM $NH_4HCO_3$ in IsPrOH) 32 mg of pure product were isolated in the form of sodium salt.
Analysis (ESI$^-$) = 276.97 (M-1)
$^1$H-NMR (300 MHz, $D_2O$): δ (ppm) 5.57 (1 H, t), 3.90 (2H, s), 3.48-3.40 (2H, m), 1.62 (3H, s).

Examples 10-16

The following compounds were obtained working in a way similar to that described in Example 7.

10) S-[2-(ethylthio)propyl]ester of the trisodium salt of thiodiphosphoric acid

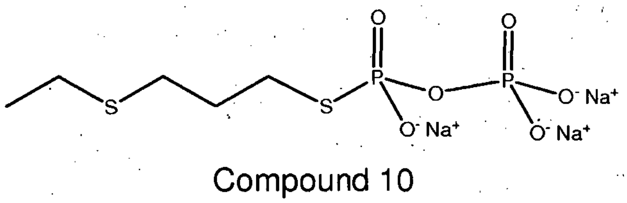

Compound 10

Analysis (ESI$^-$) = 294.96 (M-1)
$^1$H-NMR (300 MHz, $D_2O$): δ (ppm) 3.14-2.96 (2H, m), 2.81 (2H, t), 2.71 (2H, q), 2.12-2.04 (2H, m), 1.35 (3H, t).

11) S-(4-cyanobutyl)ester of the trisodium salt of thiodiphosphoric acid

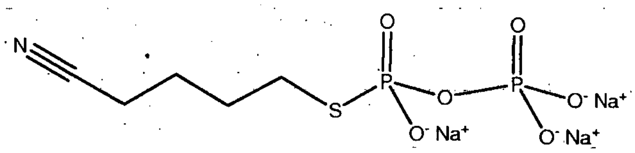

Compound 11

Analysis ($ESI^-$) = 276.1 (M-1)
$^1$H-NMR (300 MHz, $D_2O$): $\delta$ (ppm) 2.84-2.72 (2H, m), 2.45 (2H, t), 1.80-1.65 (4H, m).

12) S-(2-ethyl-1,3-dioxolanyl)ester of the trisodium salt of thiodiphosphoric acid

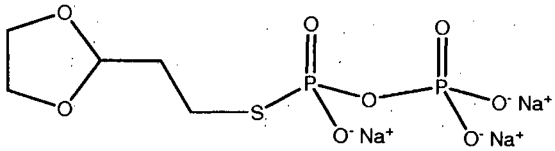

Compound 12

Analysis ($ESI^-$) = 295.1 (M-1)
$^1$H-NMR (300 MHz, $D_2O$): $\delta$ (ppm) 4.98 (1 H, t), 3.98-3.83 (4H, m), 2.87-2.77 (2H, m), 2.04-1.98 (2H, m).

13) S-(4-fluorobutyl)ester of the trisodium salt of thiodiphosphoric acid

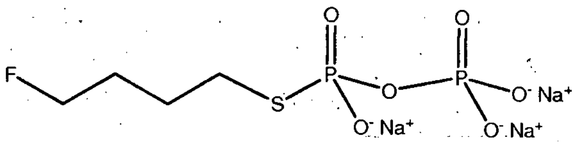

Compound 13

LCMS Analysis ($ESI^+$) = 295.1 (M+1)
$^1$H-NMR (300 MHz, $D_2O$): $\delta$ (ppm) 4.55 (1 H, t), 4.39 (1 H, t), 2.84-2.73 (2H, m), 1.84-1.64 (4H, m).

14) S-(6-heptenyl)ester of the trisodium salt of thiodiphosphoric acid

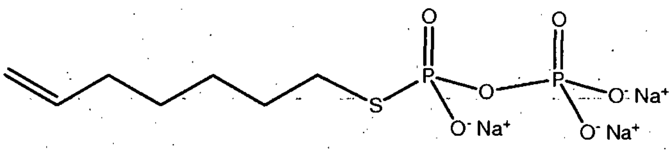

Compound 14

LCMS Analysis ($ESI^+$) = 291.1 (M+1)

$^1H$-NMR (300 MHz, $D_2O$): δ (ppm) 5.90-5.73 (1 H, m), 4.96 (1 H, d), 4.88 (1 H, d), 2.78-2.70 (2H, m), 1.99-1.96 (2H, m), 1.61-1.56 (2H, m).

15) S-[ethyl-2-tetrahydro-2*H*-2-pyranyloxy)] ester of the trisodium salt of thiodiphosphoric acid

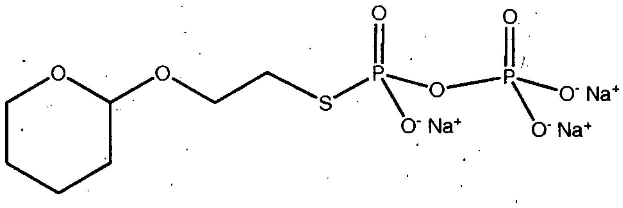

Compound 15

LCMS Analysis ($ESI^+$) = 239.1 (M+1-85)
$^1H$-NMR (300 MHz, $D_2O$): δ (ppm) 4.75 (1 H, m), 3.94-3.81 (2H, m), 3.78-3.71 (1 H, m), 3.55-3.49 (1 H, m), 3.02-2.93 (2H, m), 1.73-1.70 (2H, m), 1.48-1.46 (2H, m).

16) S-(ethyl-2-cyclohexyl)ester of the trisodium salt of thiodiphosphoric acid

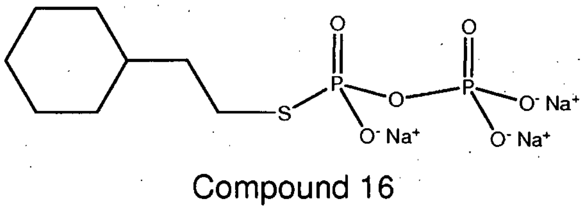

Compound 16

LCMS Analysis ($ESI^+$) = 305.1 (M+1)
$^1H$-NMR (300 MHz, $D_2O$): δ (ppm) 2.83-2.73 (2H, m), 1.64-1.43 (7H, m), 1.15-1.08 (4H, m), 0.86-0.78 (2H, m).

Example 17

Stability Tests

The following compounds were selected for the stability tests in alkaline aqueous solution in the presence of alkaline phosphatase (SIGMA Phosphatase, alkaline-Agarose from calf intestine-P0762):

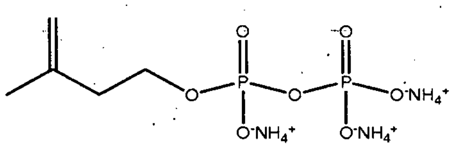

IPP

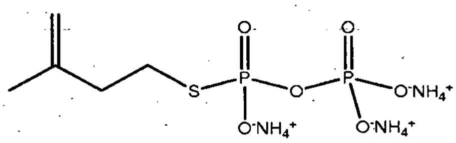

Compound 8

Solution A:

pH 8 solution: TRIS-HCl buffer and 2 mM of $MgCl_2$;

Solution B:

comparison versus solution A: 2 mM isopentenyl pyrophosphate (IPP) solution ;

Solution C:

2 mM isopentenyl thiopyrophosphate solution (Compound 8) of this invention into Solution A,

Solution D:

Solution of phosphatase enzyme immobilised on a solid substrate (agarose), 50 biological units into 10 ml of Solution A.

Experimental conditions

20 μl of Solution D were added to 0.5 ml of Solution B. The system was allowed to stand at ambient temperature under stirring. The solution was filtered to remove the enzyme and it was then analysed with HPLC-MS at predetermined time intervals (20, 30, 40, 50 and 60 minutes).

The same procedure was carried out with Solution C.

The results are shown in Table 1 below.

Table 1

| | Percentage of non-hydrolysed compound | | | | | |
|---|---|---|---|---|---|---|
| | t = 0 min | t = 20 min | t = 30 min | t = 40 min | t = 50 min | t = 60 min |
| IPP | 94% | 48% | 54% | - | - | 27% |
| Compound 8 | 100% | 90% | 90% | 90% | 90% | 90% |

The results in Table 1 show that IPP degrades rapidly while isopentenyl thiopyrophosphate (Compound 8) of this invention is very much more resistant to hydrolysis.

Example 18

Biological Tests

1. Test on peripheral blood mononucleate cells (PBMCs)

γδ T lymphocyte activation was tested by an assay involving the production of cytokines such as TNFα and IFNγ by the γδ T lymphocytes of peripheral blood mononucleate cells (PBMCs) from healthy donors (Poccia, F. et al., The Journal of Immunology 1997, 159, 6009-6017).

Mononucleate cells (PBMCs) were isolated from the peripheral blood of healthy donors, cultured at 37°C on a complete medium comprising RPMI 1640, 10% FCS, 1% L-glutamine, 1% penicillin-streptomycin in the presence of interleukin 2 (IL-2), T lymphocyte growth factor, and 5% $CO_2$ in a controlled humidity environment, and then stimulated with isopentenyl pyrophosphate (IPP 10 μM) as a positive activation control and with various doses (10 μM, 100 μM) and 1000 μM) of compounds according to the invention, respectively.

Then it was performed a cytofluorimetric labelling using specific monoclonal antibodies (membrane labelling for the expansion and intra-cytoplasm labelling for the cytokines) and a cytofluorimetric measurement in order to establish the percentage of γδ T cells producing TNFα and IFNγ (cytokine production assay).

The results are shown in Table 2 below, where "PBMC/IL-2" denotes the peripheral blood mononucleate cells from healthy donors in the presence of the complete substrate alone.

Table 2

| Percentage increase in γδ T lymphocytes producing TNFα and IFNγ | | | |
|---|---|---|---|
| Compound | Experimental conditions | % increase in γδ T lymphocytes producing | |
| | | INFγ | TNFα |
| | PBMC/IL-2 (blank) | 2-8 | 4-11 |
| IPP (std) | PBMC/IL-2/IPP (10 μM) | 100 | 100 |

(continued)

| Percentage increase in γδ T lymphocytes producing TNFα and IFNγ | | | |
|---|---|---|---|
| Compound | Experimental conditions | % increase in γδ T lymphocytes producing | |
| | | INFγ | TNFα |
| Compound 1 | PBMC/IL-2/compound 1 (10 μM) | 131 | 81 |
| | PBMC/IL-2/compound 1 (100 μM) | 94 | 127 |
| | PBMC/IL-2/compound 1 (1000 μM) | 132 | 81.5 |
| Compound 2 | PBMC/IL-2/compound 2 (10 μM) | 56.4 | 60.4 |
| | PBMC/IL-2/compound 2 (100 μM) | 67 | 71.8 |
| | PBMC/IL-2/compound 2 (1000 μM) | 97.3 | 765 |
| Compound 3 | PBMC/IL-2/compound 3 (10 μM) | - | 495 |
| | PBMC/IL-2/compound 3 (100 μM) | - | 123 |
| | PBMC/IL-2/compound 3 (1000 μM) | - | 158.4 |
| Compound 4 | PBMC/IL-2/compound 4 (10 μm) | - | 70.7 |
| | PBMC/IL-2/compound 4 (100 μM) | - | 130.4 |
| | PBMC/IL-2/compound 4 (1000 μM) | - | 299.3 |
| Compound 5 | PBMC/IL-2/compound 5 (10 μM) | - | 48 |
| | PBMC/IL-2/compound 5 (100 μM) | - | 229 |
| | PBMC/IL-2/compound 5 (1000 μM) | - | 337 |
| Compound 6 | PBMC/IL-2/compound 6 (10 μM) | - | 40 |
| | PBMC/IL-2/compound 6 (100 μM) | - | 114 |
| | PBMC/IL-2/compound 6 (1000 μM) | - | 310 |
| Compound 7 | PBMC/IL-2/compound 7 (10 μM) | 82 | 86 |
| | PBMC/IL-2/compound 7 (100 μM) | 112 | 116 |
| | PBMC/IL-2/compound 7 (1000 μM) | 95 | 87 |
| Compound 8 | PBMC/IL-2/compound 8 (10 μM) | 21 | 39 |
| | PBMC/IL-2/compound 8 (100 μM) | 865 | 92.6 |
| | PBMC/IL-2/compound 8 (1000 μM) | 182 | 174.3 |
| Compound 9 | PBMC/IL-2/compound 9 (10 μM) | - | 82.3 |
| | PBMC/IL-2/compound 9 (100 μM) | - | 175 |
| | PBMC/IL-2/compound 9 (1000 μM) | - | 464 |
| Compound 10 | PBMC/IL-2/compound 10 (10 μM) | - | - |
| | PBMC/IL-2/compound 10 (100 μM) | - | - |
| | PBMC/IL-2/compound 10 (1000 μM) | - | - |
| - = not tested | | | |

The data in Table 2 shows that all the compounds of this invention increase the number of γδ T cells producing cytokines (activation of γδ T lymphocytes); in particular Compound 9 has an activity similar to that of natural antigen IPP under the same experimental conditions (i.e. at a concentration of 10 μM), and a very clear increase at 100 and 1000 μM.

2. Peripheral blood mononucleate cell (PBMC) cellular expansion assay

The effect of the compounds of this invention on the expansion of γδ T lymphocytes was investigated through an assay carried out on γδ T lymphocytes originating from the peripheral blood mononucleate cells from healthy donors (PBMCs).

The PBMCs were separated by centrifuging (Ficoll) on whole blood from samples (residual samples) from 7 healthy donors.

After the initial percentage of Vγ9Vδ2 cells from each donor had been evaluated by labelling with an anti-Vd2-FITC antibody and cytofluorimetric analysis, the cells were placed on 24-well plates ($10^6$ cells/ml) in complete medium (having the composition mentioned in section 1 of this example) in the presence of Compound 9 of this invention (20 μM). In control experiments the cells were treated with IPP at the same concentrations and under the same Experimental conditions.

On the tenth day of culture the cells were recovered and counted by means of a living count with Trypan Blue, and labelled with the same anti-Vd2-FITC antibody, and finally tested by cytofluorimetry to evaluate the percentage of Vγ9Vδ2 cells.

The absolute number of Vγ9Vδ2 cells was calculated using the following formula:

No. of Vγ9Vδ2 cells = (total absolute no. of cells after expansion x % of Vγ9Vδ2 cells after expansion) /100.

The expansion index was calculated using the ratio between the absolute number of Vγ9Vδ2 cells after expansion and the absolute number of initial Vγ9Vδ2 cells (Poccia, F. et al., J. Immunol., 1997, 159: 6009-6017).

The results obtained are shown in Table 3 below and in Figure 1.

Table 3

| Donor | Expansion Index | |
|---|---|---|
| | Compound 9 (20 γM) | IPP (20 γM) |
| A | 129.28 | 5.41 |
| B | 80.96 | 2.42 |
| C | 5.30 | 11.11 |
| D | 59.10 | 2.81 |
| E | 624.02 | 72.92 |
| F | 35.37 | 23.87 |
| G | 42.09 | 3.35 |

3. Test on γδ T cell lines; production of TNFα and $EC_{50}$ test according to E. Espinosa et al. (J. Biol. Chem. 2001, 276, 18337-44)

3.1 Growth of the γδ T lymphocyte line

A buffy coat from a healthy donor with a high basal percentage of γδ T lymphocytes (approximately 6%) was selected for growth of the γδ T lymphocyte lines.

The PBMCs were separated out and cultured at a concentration of 1 x $10^6$ cells/ml in the presence of IPP (160 ng/ml) and IL-2 (100 U/ml).

After 7 days incubation at 37°C with 5% $CO_2$, 3 ml of medium were sampled and replaced with the same amount of fresh medium ennched with IL-2 (final concentration 100 U/ml).

After a further 7 days in the incubator an aliquot of the cultured cells was taken to label the membrane and measure the percentage of γδ T cells obtained from the expansion.

In the specific instance a cell population of 85% of γδ2 positive T cells was obtained.

This cell line was then used to evaluate the production of TNFα following stimulation of the cells with the compounds under investigation, using an ELISA test.

3.2 ELISA test for the production of TNFα

In the ELISA test there were used cells from the line produced as disclosed above in a concentration of 2.5 x $10^5$ cells/ml (5 x $10^4$ cells/well).

The cells were then stimulated with the compounds under investigation and with isopentenyl pyrophosphate (IPP) as a reference at scalar doses.

After 24 hours the supernatants from the wells containing the stimulated cells were sampled and the ELISA test was carried out using the "Human TNFα Screening Set" kit (Endogen). The kit provides for treatment with an anti-TNFα biotinylate polyclonal antibody, the addition of peroxidase conjugated with streptavidine and quantitative determination using a chromogenic substrate. The plates were read spectrophotometrically at 450 nm.

Each compound was tested in duplicate.

The results obtained were then analysed using a specific analysis program (Graph Pad Prism) and yielded the $EC_{50}$ for the compounds under investigation (Table 4).

Table 4

| $EC_{50}$ values obtained using the ELISA test for the production of TNFα in the γδ T lymphocyte line | |
|---|---|
| Compound | EC50 (μM) |
| IPP | 5.92 |
| 1 | 100 |
| 2 | 0.39 |
| 3 | 13 |
| 4 | 10 |
| 5 | 14 |
| 6 | 75 |
| 7 | 55 |
| 8 | 3.75 |
| 9 | 0.0048 |
| 10 | 0.055 |

Table 3 shows that the $EC_{50}$ values for the compounds in Examples 10 and 9 are equal to 100 and 1000 times the $EC_{50}$ for IPP, respectively.

## Claims

**1.** A compound of general formula (I):

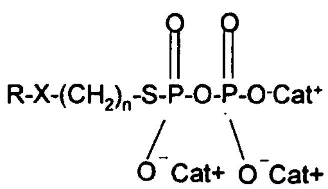

(I)

in which
X is an oxygen atom, a sulphur atom or a covalent bond,
n is zero when X is a covalent bond and is 1, 2 or 3 when X is O or S,
R is

(i) a straight or branched saturated or unsaturated aliphatic group
having 1 to 9 carbon atoms, in which one hydrogen atom may be replaced by a halogen atom, a hydroxy group, a nitrile group, a cycloalkyl group having 3 to 6 carbon atoms, an aryl group or a heterocyclic group, or
(ii) a heterocyclic group, and

$Cat^+$ is a physiologically acceptable organic cation selected from lysine, arginine, tromethamine, hydroxyethylpyrrolidine, triethanolamine and N-methylglucamine or a physiologically acceptable inorganic cation selected from alkali metals and alkaline earth metals.

2. A compound of formula (I) according to Claim 1, **characterised in that** one hydrogen atom of said straight or branched saturated or unsaturated aliphatic group having 1 to 9 carbon atoms is replaced by a halogen atom, a hydroxy group, a nitrile group, a cycloalkyl group having 3 to 6 carbon atoms, an aryl group or a heterocyclic group.

3. A compound of formula (I) according to Claim 2, **characterised in that** the halogen atom is chlorine or fluorine.

4. A compound of formula (I) according to Claim 2, **characterised in that** the aryl group is selected from the group comprising benzene and pyridine.

5. A compound of formula (I) according to Claim 2, **characterised in that** the heterocyclic group is selected from the group comprising 3-methyloxetane, dioxolane and tetrahydropyran.

6. A method for preparing a compound of formula (I)

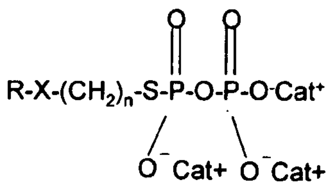

**(I)**

in which
X is an oxygen atom, a sulphur atom or a covalent bond,
n is zero when X is a covalent bond and is 1, 2 or 3 when X is O or S,
R is

(i) a straight or branched saturated or unsaturated aliphatic group
having 1 to 9 carbon atoms, in which one hydrogen atom may be replaced by a halogen atom, a hydroxy group, a nitrile group, a cycloalkyl group having 3 to 6 carbon atoms, an aryl group or a heterocyclic group, or
(ii) a heterocyclic group, and

$Cat^+$ is a physiologically acceptable organic cation selected from lysine, arginine, tromethamine, hydroxyethylpyrrolidine, triethanolamine and N-methylglucamine or a physiologically acceptable inorganic cation selected from alkali metals and alkaline earth metals,
**characterised in that** an alcohol of formula (II)

$$R\text{-}X\text{-}(CH_2)_n\text{-}OH \qquad (II)$$

where n, X and R have the above mentioned meanings, is reacted in solid phase with a thiopyrophosphate of formula (III)

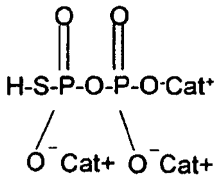

(III)

where $Cat^+$ has the above mentioned meanings.

7. A method according to Claim 6, **characterised in that** the solid support used to carry out the reaction in solid phase according to this invention is polystyrene benzene sulphonyl chloride (PS-TsCl) having a low loading capacity (1.3 mmol/g).

8. A method according to any one of Claims 6 to 7, **characterised in that** the equivalents of alcohol of formula (II) which are bound to the solid support range from 1 to 5 depending upon the nature of the R group.

9. A pharmaceutical composition comprising a compound of formula (I)

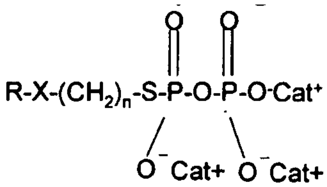

(I)

in which
X is an oxygen atom, a sulphur atom or a covalent bond,
n is zero when X is a covalent bond and is 1, 2 or 3 when X is O or S,
R is

(i) a straight or branched saturated or unsaturated aliphatic group having 1 to 9 carbon atoms, in which one hydrogen atom may be replaced by a halogen atom, a hydroxy group, a nitrile group, a cycloalkyl group having 3 to 6 carbon atoms, an aryl group or a heterocyclic group, or
(ii) a heterocyclic group, and

$Cat^+$ is a physiologically acceptable organic cation selected from lysine, arginine, tromethamine, hydroxyethylpyrrolidine, triethanolamine and N-methylglucamine or a physiologically acceptable inorganic cation selected from alkali metals and alkaline earth metals,
and at least one physiologically acceptable carrier.

10. A compound for use in activating mammalian γδ T lymphocytes **characterised in that** said compound is represented by the following formula I

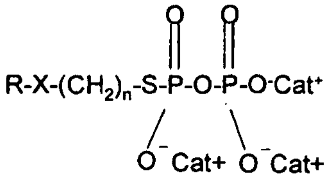

(I)

in which
X is an oxygen atom, a sulphur atom or a covalent bond,
n is zero when X is a covalent bond and is 1, 2 or 3 when X is O or S,
R is

(i) a straight or branched saturated or unsaturated aliphatic group having 1 to 9 carbon atoms, in which one hydrogen atom may be replaced by a halogen atom, a hydroxy group, a nitrile group, a cycloalkyl group having 3 to 6 carbon atoms, an aryl group or a heterocyclic group, or
(ii) a heterocyclic group, and

$Cat^+$ is a physiologically acceptable organic cation selected from lysine, arginine, tromethamine, hydroxyethylpyrrolidine, triethanolamine and N-methylglucamine or a physiologically acceptable inorganic cation selected from ammonium, alkali metals and alkaline earth metals.

**11.** A compound for use according to Claim 10, **characterised in that** said use comprises

- sampling a suitable amount of peripheral blood from a patient,
- contacting the said blood with said compound of formula I for sufficient time to stimulate ex *vivo* proliferation of γδ T lymphocytes,
- collecting the thus treated γδ T cells, and
- administering them to the patient by intravenous route.

**Patentansprüche**

**1.** Verbindung der allgemeinen Formel (I):

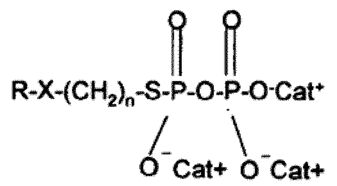

**(I)**

in welcher
X ein Sauerstoffatom, ein Schwefelatom oder eine kovalente Bindung ist, n Null ist, wenn X eine kovalente Bindung ist, und 1, 2 oder 3 ist, wenn X O oder S ist,
R

(i) eine gerade oder verzweigte gesättigte oder ungesättigte aliphatische Gruppe ist, die 1 bis 9 Kohlenstoffatome besitzt, in welcher ein Wasserstoffatom ersetzt sein kann durch ein Halogenatom, eine Hydroxygruppe, eine Nitrilgruppe, eine Cycloalkylgruppe, die 3 bis 6 Kohlenstoffatome besitzt, eine Arylgruppe oder heterocyclische Gruppe, oder
(ii) eine heterocyclische Gruppe ist, und

$Cat^+$ ein physiologisch verträgliches organisches Kation ist, ausgewählt aus Lysin, Arginin, Tromethamin, Hydroxyethylpyrrolidin, Triethanolamin und N-Methylglucamin, oder ein physiologisch verträgliches anorganisches Kation ist, ausgewählt aus Alkalimetallen oder Erdalkalimetallen.

**2.** Verbindung nach Formel (I) gemäß Anspruch 1, **dadurch gekennzeichnet, dass** ein Wasserstoffatom der genannten geraden oder verzweigten gesättigten oder ungesättigten aliphatischen Gruppe, die 1 bis 9 Kohlenstoffatome besitzt, ersetzt ist durch ein Halogenatom, eine Hydroxygruppe, eine Nitrilgruppe, eine Cycloalkylgruppe, die 3 bis 6 Kohlenstoffatome besitzt, eine Arylgruppe oder eine heterocyclische Gruppe.

**3.** Verbindung nach Formel (I) gemäß Anspruch 2, **dadurch gekennzeichnet, dass** das Halogenatom Chlor oder Fluor ist.

**4.** Verbindung nach Formel (I) gemäß Anspruch 2, **dadurch gekennzeichnet, dass** die Arylgruppe ausgewählt ist aus der Gruppe, die Benzol und Pyridin umfasst.

**5.** Verbindung nach Formel (I) gemäß Anspruch 2, **dadurch gekennzeichnet, dass** die heterocyclische Gruppe ausgewählt ist aus der Gruppe, welche 3-Methyloxetan, Dioxolan und Tetrahydropyran umfasst.

**6.** Verfahren zur Herstellung einer Verbindung der Formel (I)

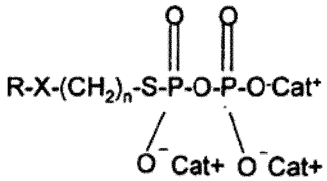

**(I)**

in welcher
X ein Sauerstoffatom, ein Schwefelatom oder eine kovalente Bindung ist,
n Null ist, wenn X eine kovalente Bindung ist, und 1, 2 oder 3 ist, wenn X O oder S ist,
R

(i) eine gerade oder verzweigte gesättigte oder ungesättigte aliphatische Gruppe ist, die 1 bis 9 Kohlenstoffatome besitzt, in welcher ein Wasserstoffatom ersetzt sein kann durch ein Halogenatom, eine Hydroxygruppe, eine Nitrilgruppe, eine Cycloalkylgruppe, die 3 bis 6 Kohlenstoffatome besitzt, eine Arylgruppe oder heterocyclische Gruppe, oder
(ii) eine heterocyclische Gruppe ist, und

$Cat^+$ ein physiologisch verträgliches organisches Kation ist, ausgewählt aus Lysin, Arginin, Tromethamin, Hydroxyethylpyrrolidin, Triethanolamin und N-Methylglucamin, oder ein physiologisch verträgliches anorganisches Kation ist, ausgewählt aus Alkalimetallen oder Erdalkalimetallen,
**dadurch gekennzeichnet, dass** ein Alkohol der Formel (II)

$$R\text{-}X\text{-}(CH_2)_n\text{-}OH \qquad (II)$$

worin n, X und R die oben genannten Bedeutungen haben,
in fester Phase mit einem Thiopyrophosphat der Formel (III)

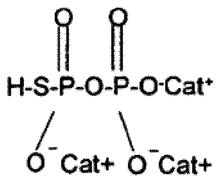

**(III)**

umgesetzt wird,
worin $Cat^+$ die oben genannten Bedeutungen hat.

**7.** Verfahren gemäß Anspruch 6, **dadurch gekennzeichnet, dass** der feste Träger, der verwendet wird, um die Reaktion in fester Phase gemäß dieser Erfindung auszuführen, Polystyrolbenzensulfonylchlorid (PS-TsCl) ist, das eine geringe Ladekapazität (1,3 mmol/g) besitzt.

**8.** Verfahren gemäß einem der Ansprüche 6 bis 7, **dadurch gekennzeichnet, dass** die Äquivalente des Alkohols der Formel (II), welche an den festen Träger gebunden sind, von 1 bis 5 reichen, abhängig von der Natur der Gruppe R.

**9.** Pharmazeutische Zusammensetzung, umfassend eine Verbindung der Formel (I)

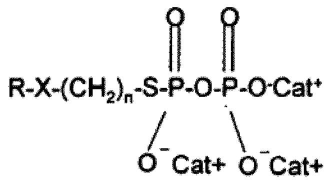

(I)

in welcher
X ein Sauerstoffatom, ein Schwefelatom oder eine kovalente Bindung ist,
n Null ist, wenn X eine kovalente Bindung ist, und 1, 2 oder 3 ist, wenn X O oder S ist,
R

(i) eine gerade oder verzweigte gesättigte oder ungesättigte aliphatische Gruppe ist, die 1 bis 9 Kohlenstoffatome besitzt, in welcher ein Wasserstoffatom ersetzt sein kann durch ein Halogenatom, eine Hydroxygruppe, eine Nitrilgruppe, eine Cycloalkylgruppe, die 3 bis 6 Kohlenstoffatome besitzt, eine Arylgruppe oder heterocyclische Gruppe, oder
(ii) eine heterocyclische Gruppe ist, und

$Cat^+$ ein physiologisch verträgliches organisches Kation ist, ausgewählt aus Lysin, Arginin, Tromethamin, Hydroxyethylpyrrolidin, Triethanolamin und N-Methylglucamin, oder ein physiologisch verträgliches anorganisches Kation ist, ausgewählt aus Alkalimetallen oder Erdalkalimetallen,
und wenigstens einen physiologisch verträglichen Träger.

**10.** Verbindung zu Verwendung bei der Aktivierung von Säugetier-γδ-T-Lymphozyten, **dadurch gekennzeichnet, dass** die genannte Verbindung durch die folgende Formel I dargestellt wird,

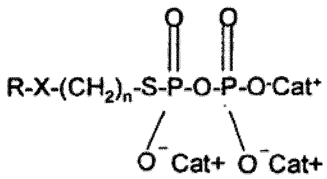

(I)

in welcher
X ein Sauerstoffatom, ein Schwefelatom oder eine kovalente Bindung ist,
n Null ist, wenn X eine kovalente Bindung ist, und 1, 2 oder 3 ist, wenn X O oder S ist,
R

(i) eine gerade oder verzweigte gesättigte oder ungesättigte aliphatische Gruppe ist, die 1 bis 9 Kohlenstoffatome besitzt, in welcher ein Wasserstoffatom ersetzt sein kann durch ein Halogenatom, eine Hydroxygruppe, eine Nitrilgruppe, eine Cycloalkylgruppe, die 3 bis 6 Kohlenstoffatome besitzt, eine Arylgruppe oder heterocyclische Gruppe, oder
(ii) eine heterocyclische Gruppe ist, und

$Cat^+$ ein physiologisch verträgliches organisches Kation ist, ausgewählt aus Lysin, Arginin, Tromethamin, Hydroxyethylpyrrolidin, Triethanolamin und N-Methylglucamin, oder ein physiologisch verträgliches anorganisches Kation ist, ausgewählt aus Alkalimetallen oder Erdalkalimetallen.

**11.** Verbindung zur Verwendung gemäß Anspruch 10, **dadurch gekennzeichnet, dass** die genannte Verwendung umfasst

- die Probenahme einer geeigneten Menge peripheren Blutes eines Patienten,
- das in Kontakt Bringen des genannten Blutes mit der genannten Verbindung der Formel I einen Zeitraum lang, der ausreicht, um die *ex vivo* Proliferation von γδ-T-Lymphozyten zu stimulieren,
- Sammeln der dadurch behandelten γδ-T-Zellen und
- deren Verabreichen an einen Patienten auf intravenösem Weg.

## Revendications

**1.** Composé de formule générale (I) :

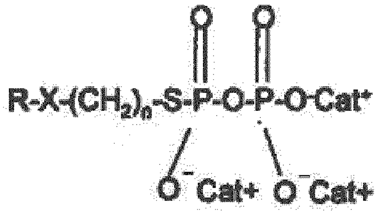

(I)

dans laquelle
X est un atome d'oxygène, un atome de soufre ou une liaison covalente,
n vaut zéro quand X est une liaison covalente et vaut 1, 2 ou 3 quand X est O ou S,
R est

(i) un groupe aliphatique linéaire ou ramifié, saturé ou insaturé, ayant de 1 à 9 atomes de carbone, dans lequel un atome d'hydrogène peut être remplacé par un atome d'halogène, un groupe hydroxy, un groupe nitrile, un groupe cycloalkyle ayant de 3 à 6 atomes de carbone, un groupe aryle ou un groupe hétérocyclique, ou
(ii) un groupe hétérocyclique, et

$Cat^+$ est un cation organique physiologiquement acceptable choisi parmi la lysine, l'arginine, la trométhamine, l'hydroxyéthylpyrrolidine, la triéthanolamine et la N-méthylglucamine, ou un cation inorganique physiologiquement acceptable choisi parmi les métaux alcalins et les métaux alcalino-terreux.

**2.** Composé de formule (I) selon la revendication 1, **caractérisé en ce qu'**un atome d'hydrogène dudit groupe aliphatique linéaire ou ramifié, saturé ou insaturé, ayant de 1 à 9 atomes de carbone, est remplacé par un atome d'halogène, un groupe hydroxy, un groupe nitrile, un groupe cycloalkyle ayant de 3 à 6 atomes de carbone, un groupe aryle ou un groupe hétérocyclique.

**3.** Composé de formule (I) selon la revendication 2, **caractérisé en ce que** l'atome d'halogène est le chlore ou le fluor.

**4.** Composé de formule (I) selon la revendication 2, **caractérisé en ce que** le groupe aryle est choisi dans l'ensemble comprenant le benzène et la pyridine.

**5.** Composé de formule (I) selon la revendication 2, **caractérisé en ce que** le groupe hétérocyclique est choisi dans l'ensemble comprenant le 3-méthyloxétane, le dioxolane et le tétrahydropyrane.

**6.** Procédé pour préparer un composé de formule (I) :

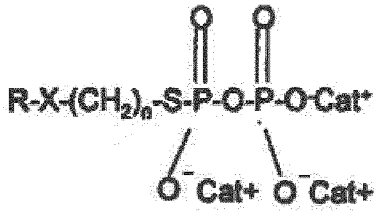

(I)

dans laquelle
X est un atome d'oxygène, un atome de soufre ou une liaison covalente,
n vaut zéro quand X est une liaison covalente et vaut 1, 2 ou 3 quand X est O ou S,
R est

(i) un groupe aliphatique linéaire ou ramifié, saturé ou insaturé, ayant de 1 à 9 atomes de carbone, dans lequel un atome d'hydrogène peut être remplacé par un atome d'halogène, un groupe hydroxy, un groupe nitrile, un groupe cycloalkyle ayant de 3 à 6 atomes de carbone, un groupe aryle ou un groupe hétérocyclique, ou
(ii) un groupe hétérocyclique, et

$Cat^+$ est un cation organique physiologiquement acceptable choisi parmi la lysine, l'arginine, la trométhamine, l'hydroxyéthylpyrrolidine, la triéthanolamine et la N-méthylglucamine, ou un cation inorganique physiologiquement acceptable choisi parmi les métaux alcalins et les métaux alcalino-terreux,
**caractérisé en ce qu'**un alcool de formule (II) :

$$R\text{-}X\text{-}(CH_2)_n\text{-}OH \qquad (II)$$

dans laquelle n, X et R ont les significations mentionnées ci-dessus,
est mis à réagir en phase solide avec un thiopyrophosphate de formule (III) :

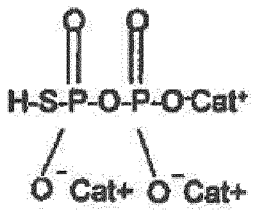

(III)

dans laquelle $Cat^+$ a les significations mentionnées ci-dessus.

7. Procédé selon la revendication 6, **caractérisé en ce que** le support solide utilisé pour la mise en oeuvre de la réaction en phase solide selon cette invention est le polystyrène-chlorure de benzènesulfonyle (PS-TsCl) ayant une faible capacité de chargement (1,3 mmol/g).

8. Procédé selon l'une quelconque des revendications 6 et 7, **caractérisé en ce que** les équivalents d'alcool de formule (II) qui sont liés au support solide sont situés dans la plage allant de 1 à 5 en fonction de la nature du groupe R.

9. Composition pharmaceutique comprenant un composé de formule (I) :

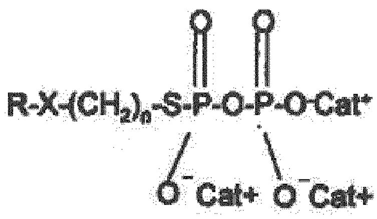

(I)

dans laquelle
X est un atome d'oxygène, un atome de soufre ou une liaison covalente,
n vaut zéro quand X est une liaison covalente et vaut 1, 2 ou 3 quand X est O ou S,
R est

(i) un groupe aliphatique linéaire ou ramifié, saturé ou insaturé, ayant de 1 à 9 atomes de carbone, dans lequel un atome d'hydrogène peut être remplacé par un atome d'halogène, un groupe hydroxy, un groupe nitrile, un groupe cycloalkyle ayant de 3 à 6 atomes de carbone, un groupe aryle ou un groupe hétérocyclique, ou
(ii) un groupe hétérocyclique, et

$Cat^+$ est un cation organique physiologiquement acceptable choisi parmi la lysine, l'arginine, la trométhamine, l'hydroxyéthylpyrrolidine, la triéthanolamine et la N-méthylglucamine, ou un cation inorganique physiologiquement

acceptable choisi parmi les métaux alcalins et les métaux alcalino-terreux,
et au moins un véhicule physiologiquement acceptable.

**10.** Composé pour utilisation dans l'activation des lymphocytes T $\gamma\delta$ de mammifère, **caractérisé en ce que** ledit composé est représenté par la formule I suivante :

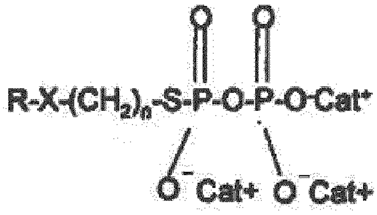

(I)

dans laquelle
X est un atome d'oxygène, un atome de soufre ou une liaison covalente,
n vaut zéro quand X est une liaison covalente et vaut 1, 2 ou 3 quand X est O ou S,
R est

(i) un groupe aliphatique linéaire ou ramifié, saturé ou insaturé, ayant de 1 à 9 atomes de carbone, dans lequel un atome d'hydrogène peut être remplacé par un atome d'halogène, un groupe hydroxy, un groupe nitrile, un groupe cycloalkyle ayant de 3 à 6 atomes de carbone, un groupe aryle ou un groupe hétérocyclique, ou
(ii) un groupe hétérocyclique, et

$Cat^+$ est un cation organique physiologiquement acceptable choisi parmi la lysine, l'arginine, la trométhamine, l'hydroxyéthylpyrrolidine, la triéthanolamine et la N-méthylglucamine, ou un cation inorganique physiologiquement acceptable choisi parmi l'ammonium, les métaux alcalins et les métaux alcalino-terreux.

**11.** Composé pour utilisation selon la revendication 10, **caractérisé en ce que** ladite utilisation comprend

- l'échantillonnage d'une quantité convenable de sang périphérique auprès d'un patient,
- la mise en contact dudit sang avec ledit composé de formule I pendant un temps suffisant pour stimuler la prolifération ex vivo de lymphocytes T $\gamma\delta$,
- la collecte des cellules T $\gamma\delta$ ainsi traitées, et
- leur administration au patient par voie intraveineuse.

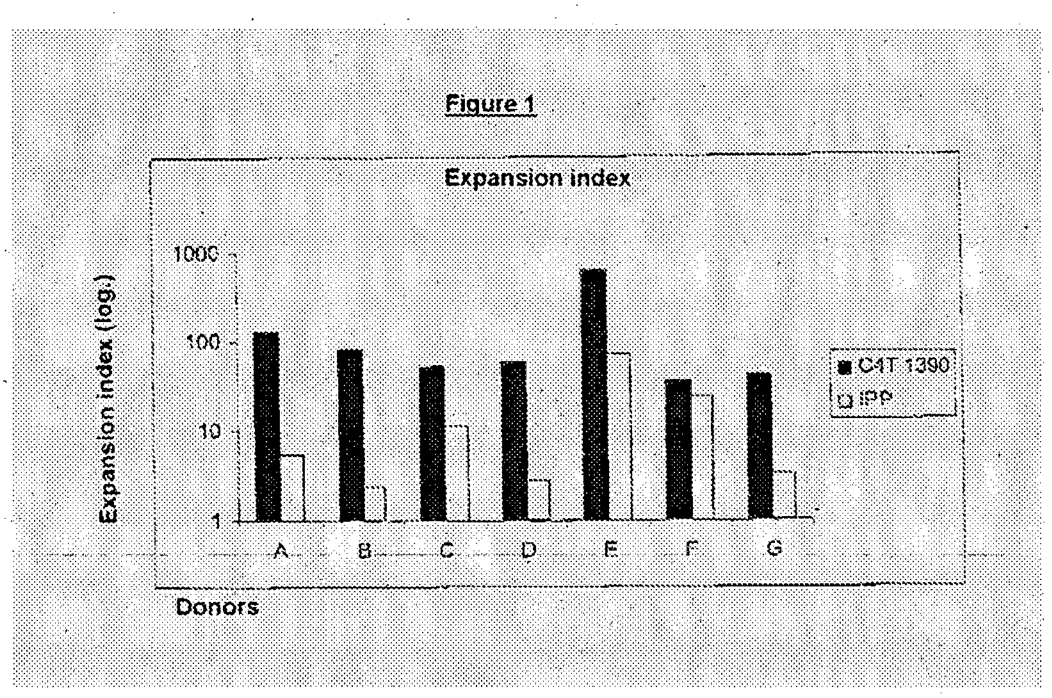
Figure 1
Expansion index
Expansion index (log.)
1000
100
10
1
A
B
C
D
E
F
G
C1T 1390
IPP
Donors

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5639653 A
- US 6660723 B1
- US 6624151 B1, Belmant


### Non-patent literature cited in the description


- **FOURNIÉ et al.** *Microbes and Infection,* 2001, vol. 3, 645-654
- **BENDELAC et al.** *Nat. Rev. Immunol.,* 2001, vol. 1, 177-185
- **WANG et al.** *J. Clin. Invest,* 2001, vol. 108, 1349-1357
- **GIRARDI et al.** *Science,* 2001, vol. 294, 605-609
- **SICARD et al.** *Mol. Med.,* 2001, vol. 7, 711-722
- **LAFONT, V. et al.** Isopentenyl Pyrophosphate, a Mycoobacterial Non-peptidic Antigen, Triggers Delayed and Highly Sustained Signaling in Human γδ T Lymphocytes without Inducing Down-modulation of T Cell Antigen Receptor. *J. Biol. Chem.,* 2001, vol. 276, 15961-15967
- **ESPINOSA, E. et al.** *J. Biol. Chem.,* 2001, vol. 276, 18337-18344
- **HINTZ, M. et al.** *Febs. Lett.,* 2001, vol. 509, 317-322
- **MORITA, C. et al.** *J. Immunol.,* 2001, vol. 167, 36-41
- **BELMANT, C. et al.** *FASEB J.,* 2000, vol. 14, 1669-1670
- *Bioorg. & Med. Chem. Lett.,* 2003, vol. 13, 1257-1260
- **ZGANI, I. et al.** *J. Med. Chem.,* 2004, vol. 47, 4600-4612
- **PHAN, R.M. ; POULTER, D.** *J. Org. Chem.,* 2001, vol. 66, 6705-6710
- **CHEN et al.** *J. Biol. Chem.,* 2002, vol. 277, 7369-7376
- **DALE POULTER et al.** *J. Am. Chem. Soc.,* 1991, vol. 113, 4895
- *Organic Letters,* 2000, vol. 15, 2287
- **POULTER, D.C. ; PHAN, R.M.** *Organic Letters,* 2000, vol. 2, 2287-2289
- **POULTER.** *J. Org. Chem.,* 2001, vol. 66, 6705-6710
- **POCCIA, F. et al.** *J. Immunol.,* vol. 159, 6009-6017
- **WALLACE, M. et al.** *Clin. Exp. Immunol.,* vol. 103, 177-184
- **POCCIA, F. et al.** *J. Infect. Dis.,* vol. 180, 858-861
- **AGERBERTH, B. et al.** *Blood,* vol. 96, 3086-3093
- **DIELI, F. et al.** *J. Infect. Dis.,* vol. 184, 1082-1085
- **DIELI F. et al.** *Eur. J. Immunol.,* vol. 30, 1512-1519
- **WIHELMM, M. et al.** *Blood,* vol. 102, 200-206
- **KUNZMANN, V. et al.** *Blood,* vol. 96, 384-392
- **FISCH, P. et al.** *Eur. J. Immunol.,* vol. 27, 3368-3379
- **SICARD, H.T. et al.** *Mol. Med.,* vol. 7, 711-722
- **VIEY, E. et al.** *J. Immunol.,* 2005, vol. 174, 1338-1347
- **AMSLINGER et al.** *J. Org. Chem.,* 2002, vol. 67, 4590
- **POCCIA, F. et al.** *The Journal of Immunology,* 1997, vol. 159, 6009-6017
- **POCCIA, F. et al.** *J. Immunol.,* 1997, vol. 159, 6009-6017